# FASCICULE DE BREVET EUROPEEN

(11) **EP 2 639 845 B1**
(45) Date de publication et mention de la délivrance du brevet: **19.11.2014**
(21) Numéro de dépôt: 13152208.8
(22) Date de dépôt: 22.01.2013
(51) Int. Cl.: H01L 41/113, A61N 1/378, A61N 1/36

(54) **Capsule intracorporelle autonome à récupération d'énergie piézoélectrique**
Autonome intrakorporelle Kapsel mit piezoelektrischer Energierückgewinnung
Autonomous intracorporeal capsule with piezoelectric energy recovery

(30) Priorité: 12.03.2012 FR 1252217
(43) Date de publication de la demande: 18.09.2013
(73) Titulaire: Sorin CRM SAS, 92140 Clamart (FR)
(72) Inventeur: Deterre, Martin, 75013 Paris (FR); Lefeuvre, Elie, 93100 Montreuil (FR)
(74) Mandataire: Dupuis-Latour, Dominique

(56) Documents cités:
- US-A1- 2009 216 292
- US-A1- 2010 317 978
- US-A1- 2011 275 947
- CHANGKI MO ET AL: "Experimental validation of energy harvesting performance for pressure-loaded piezoelectric circular diaphragms;Experimental validation of energy harvesting performance for pressure-loaded piezoelectric circular diaphragms", SMART MATERIALS AND STRUCTURES, IOP PUBLISHING LTD., BRISTOL, GB, vol. 19, no. 7, 1 juin 2010 (2010-06-01), page 75010, XP020194607, ISSN: 0964-1726, DOI: 10.1088/0964-1726/19/7/075010

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation et/ou de défibrillation en cas de trouble du rythme détecté par l'appareil. Elle inclut aussi les appareils neurologiques, les implants cochléaires, etc., ainsi que les dispositifs de mesure de pH ou encore d'impédance intracorporelle, telle que mesure d'impédance transpulmonaire ou d'impédance intracardiaque.

L'invention concerne plus particulièrement ceux de ces dispositifs qui mettent en oeuvre des capsules autonomes implantées et dépourvues de toute liaison physique à un dispositif principal implanté, tel qu'un boîtier de générateur d'impulsions de stimulation.

Ces capsules autonomes sont dénommées pour cette raison "capsules *leadless",* pour les distinguer des électrodes ou des capteurs disposés à l'extrémité distale d'une sonde (*lead*), cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces capsules *leadless* peuvent être par exemple des capsules épicardiques, fixées à la paroi extérieure du coeur, ou bien des capsules endocavitaires, fixées à la paroi intérieure d'une cavité ventriculaire ou auriculaire au moyen d'une vis d'ancrage saillante, prolongeant axialement le corps de la capsule et destinée à pénétrer dans le tissu cardiaque par vissage au site d'implantation.

Une telle capsule comprend des circuits de détection/stimulation pour recueillir des potentiels de dépolarisation du myocarde et/ou pour appliquer des impulsions de stimulation au site où est implantée la capsule. La capsule porte alors une électrode appropriée qui peut être notamment constituée par une partie active de la vis d'ancrage.

Elle peut également incorporer, en variante ou en complément, un ou plusieurs capteurs permettant de mesurer localement la valeur d'un paramètre tel que le niveau d'oxygène dans le sang, la pression cardiaque endocavitaire, l'accélération de la paroi cardiaque, l'accélération du patient comme indicateur de l'activité, etc. Bien entendu, les capsules *leadless* incorporent également des moyens émetteurs/récepteurs de communication sans fil pour l'échange de données à distance.

L'invention n'est toutefois pas limitée à un type particulier de capsule, et elle est applicable indifféremment à tout type de capsule *leadless,* quelle que soit sa destination fonctionnelle.

Quelle que soit la technique mise en oeuvre, le traitement des signaux au sein de la capsule et leur transmission à distance nécessite une énergie non négligeable par rapport aux ressources énergétiques que peut stocker cette capsule. Or, compte tenu de son caractère autonome, la capsule ne peut faire appel qu'à ses ressources propres telles qu'un circuit de récupération d'énergie du mouvement de la capsule, associé à une petite batterie tampon intégrée.

La gestion de l'énergie disponible est donc un point crucial pour le développement des techniques de capsules autonomes *leadless,* en particulier leur aptitude à disposer d'un système d'auto-alimentation intégré.

De nombreuses techniques de récupération d'énergie ont été proposées, adaptées à des implants autonomes de type *leadless.*

Un grand nombre de récupérateurs d'énergie actuels sont basés sur un dispositif inertiel, c'est-à-dire qui récupère l'accélération du milieu ambiant pour agir sur une masse, dite "masse sismique", dont le déplacement relatif génère de l'électricité par l'intermédiaire d'un transducteur piézoélectrique, électromagnétique, ou électrostatique. La puissance récupérée dépend principalement de la fréquence d'excitation, de son amplitude, et de la masse inertielle. Si le spectre de l'excitation est centré sur une fréquence spécifique fixe, le récupérateur peut être conçu pour résonner à la même fréquence et bénéficier ainsi d'une amplification mécanique pour récupérer un maximum de l'énergie inertielle, mais reste limité par la course de la masse. C'est pourquoi les récupérateurs inertiels habituels sont performants uniquement pour des applications à des fréquences élevées stables et à petites amplitudes, dans le domaine industriel par exemple.

Dans le cas de l'environnement du corps humain, les excitations proviennent de l'accélération du corps ou des organes. Elles n'ont pas de fréquences spécifiques stables pour lesquelles le récupérateur d'énergie peut être optimisé de manière résonante, et ces fréquences sont très basses, inférieures à 10Hz, ce qui génère de grands déplacements et n'est pas adapté à la miniaturisation. Les générateurs existants miniaturisés adaptés au corps humain ne sont donc pas résonants, et ne peuvent générer une densité de puissance supérieure à celle des piles actuelles. Certains dispositifs non inertiels tentent de récupérer non pas l'accélération du corps ou des organes, mais leur déplacement. C'est le cas des systèmes de montres à remontage automatique qui, montés par exemple sur un implant cardiaque, restent trop volumineux et ne fournissent pas assez d'énergie, ou du cas spécifique des générateurs piézoélectriques montés sous les talons, fournissant une grande puissance mais aux dimensions macroscopiques.

Une technique alternative de récupération d'énergie pour les implants en milieu fluidique et notamment en milieu sanguin consiste à récupérer les variations de pression du fluide par une membrane flexible dont la déformation ou le mouvement peut générer de l'électricité via un transducteur. Plusieurs méthodes de transduction ont été proposées comme l'entraînement d'un rotor, produisant de l'électricité électromagnétique à la manière d'un alternateur classique. La complexité de l'agencement des pièces nécessaires à un tel système en limite largement la miniaturisation. En outre, ce type de transducteur, de part de sa nature magnétique, n'est pas compatible avec les systèmes d'imagerie par résonance magnétique (IRM).

Il a été également proposé, en plus d'un récupérateur inertiel classique basé sur le mouvement de la paroi cardiaque, un récupérateur des efforts de pression subis dans une cavité cardiaque, où la membrane flexible entraîne un générateur résonant à une plus grande fréquence que la fréquence d'excitation. Le fait que le générateur soit résonant à haute fréquence permet de récupérer une large partie de l'énergie mécanique apportée par la membrane, mais la conversion de fréquence pose des problèmes soit de fiabilité mécanique, soit de compatibilité IRM en cas de couplage par aimants. De plus, les vibrations à haute fréquence multiplient le nombre de cycles, ce qui peut s'avérer critique pour la fatigue mécanique des systèmes.

Dans Experimental Validation of Energy Harvesting Performance for Pressure-Loaded Piezoelectric Circular Diaphragms, Smart Mater. Struct., 19 (2010), 075010, Changki et al. proposent, pour récupérer la pression sanguine, d'utiliser un diaphragme piézoélectrique circulaire qui en se déformant génère de l'énergie électrique. Cependant, comme l'énergie mécanique récupérable est proportionnelle au déplacement, un diaphragme plein et très rigide ne peut pas se déformer suffisamment, sous l'effet de forces extérieures telles que celles produites par les variations de pression, pour récupérer beaucoup d'énergie mécanique. De plus, la rigidité de tels systèmes augmentant très fortement avec la réduction de la surface, leur miniaturisation est très difficile.

Le US 2009/0216292 A1 décrit un récupérateur d'énergie implantable en forme de ruban flexible, destiné à être pris en sandwich entre deux couches adjacentes de tissus corporels de manière à subir les déformations subies localement par ces tissus. Les tissus en question peuvent notamment être ceux d'un muscle de la région pectorale, à proximité du générateur d'un stimulateur cardiaque à alimenter au moyen de ce dispositif. Des fibres piézoélectriques disposées à l'intérieur du ruban se déforment comme celui-ci, entrainant la formation de charges électriques qui sont recueillies par des électrodes et récupérées dans un condensateur. Dans le cas de l'invention, il s'agit d'une configuration différente, dans la mesure où le récupérateur d'énergie n'est pas pris en sandwich dans des tissus musculaires, mais entièrement immergé dans un fluide corporel (le sang) soumis à des variations régulières de pression. Du point de vue de la stricte configuration mécanique, le récupérateur de l'invention n'est adjacent qu'à un seul tissu, typiquement la paroi du myocarde auquel il est fixé ; la capsule n'est retenue à cette paroi que par une attache souple et "flotte" librement dans la cavité cardiaque au gré des mouvements de la masse sanguine.

Le but de l'invention est de proposer un générateur d'alimentation électrique pour une capsule autonome implantable et entièrement immergeable dans un fluide corporel soumis à des variations régulières de pression, qui pallie les inconvénients ci-dessus et réponde aux impératifs de:
- récupération optimale de l'énergie induite par les variations de pression du fluide corporel ;
- miniaturisation : compatibilité avec le volume extrêmement réduit (quelques millimètres cubes) d'un implant *leadless* ;
- fiabilité : garantie de fonctionnement sur plusieurs années de durée de vie de l'implant ;
- insensibilité aux phénomènes magnétiques : notamment pour assurer la compatibilité IRM qui est aujourd'hui requise pour les dispositifs implantés ; et
- biocompatibilité : absence d'éléments extérieurs risquant de provoquer des réactions inflammatoires.

Essentiellement, l'invention propose un système d'alimentation par récupération d'énergie incorporé à une capsule implantable dont le corps de boîtier possède un élément déformable sous l'effet des variations de pression du milieu environnant, typiquement les variations de pression du sang au cours du cycle cardiaque. La déformation de cet élément est transmise à un transducteur piézoélectrique convertissant directement l'énergie mécanique de déformation en énergie électrique qui est ensuite délivrée à un module de gestion électrique et de stockage alimentant la capsule en énergie.

On notera que le système n'a besoin ni d'être résonant ni de contenir des éléments magnétiques.

Plus précisément, la capsule intracorporelle autonome est d'un type général tel que celui divulgué par le US 2009/0216292 A1 précité, c'est-à-dire comprenant un transducteur de récupération d'énergie et un module de stockage et de gestion d'énergie. Le transducteur de récupération d'énergie convertit une sollicitation physique extérieure appliquée à la capsule en grandeur électrique, et ce transducteur comprend au moins un composant piézoélectrique couplé à un élément mobile d'actionnement recevant ladite sollicitation physique extérieure. Le composant piézoélectrique présente au moins une structure à au moins une bande piézoélectrique fixée au corps de la capsule à une extrémité et apte à être soumise à une sollicitation physique extérieure en un point d'application situé à une autre extrémité. Le module de stockage et de gestion d'énergie est alimenté par le transducteur de récupération d'énergie sous l'effet d'une déformation du composant piézoélectrique due à la sollicitation physique extérieure transmise par l'élément mobile d'actionnement.

De façon caractéristique de l'invention, l'élément mobile d'actionnement comprend une surface rigide couplée au composant piézoélectrique et un soufflet élastiquement déformable de liaison de cette surface rigide au reste du corps.

Ainsi, l'invention consiste en une capsule intracorporelle autonome comprenant un récupérateur d'énergie possédant un élément mobile d'actionnement susceptible de se déformer et/ou de se déplacer par rapport au corps rigide et fixe de la capsule. Lorsque que cette dernière est placée dans une cavité cardiaque ou en milieu sanguin, la pression sanguine va s'appliquer sur le corps et l'élément mobile : du fait des variations de pression au cours du cycle cardiaque, l'élément mobile de la capsule va être soumis à des forces variables au cours du temps, et, compte tenu de sa souplesse, va se déformer ou se déplacer suivant le cycle des variations de pression, emmagasinant ainsi de l'énergie mécanique.

Cette énergie mécanique est transformée en électricité par un transducteur piézoélectrique fixé d'une part à une partie fixe du corps, et d'autre part à l'élément mobile d'actionnement. Le transducteur est ainsi également assujetti à des déformations mécaniques qu'il convertit en énergie électrique par effet piézoélectrique direct. Ce composant piézoélectrique fonctionne donc en régime forcé non résonant à la même fréquence que les variations de pression externe, il n'est donc pas soumis aux contraintes de conversion de fréquence ou de présence de masse sismique, en plus de réduire le nombre de cycles de fonctionnement.

Le composant piézoélectrique est avantageusement structuré en bandes afin de réduire sa raideur et augmenter sa déformation pour une meilleure récupération d'énergie. La structure de base est une bande longue et fine, formant une poutre encastrée à une extrémité et libre l'autre extrémité soumise à la sollicitation extérieure.

Outre la forme rectiligne, l'invention propose un certain nombre de dispositions avantageuses visant à augmenter la longueur de la structure en bandes du composant piézoélectrique tout en conservant une surface réduite.

Il est prévu notamment que la bande piézoélectrique est enroulée en spirale ou formée de segments rectilignes repliés ou encore qu'elle a une structure annulaire.

De même, au niveau de la structure du composant piézoélectrique lui-même et de manière à ajuster au mieux la raideur du système tout en gardant un système compact, le composant piézoélectrique comprend avantageusement deux bandes piézoélectriques en parallèle autour d'un point d'application commun. Le composant piézoélectrique peut également présenter une pluralité de structures de bandes disposées en parallèle. Enfin, la capsule selon l'invention peut comporter une pluralité de composants piézoélectriques disposés en série ou en parallèle

On va maintenant décrire divers exemples de mise en oeuvre de l'invention, en référence aux dessins annexés où les mêmes références numériques désignent d'une figure à l'autre des éléments identiques ou fonctionnellement semblables.
La Figure 1 est un schéma d'un ensemble de dispositifs médicaux comprenant des capsules *leadless,* implantées au sein du corps d'un patient.
La Figure 2 est un schéma par blocs fonctionnels montrant les différents circuits électroniques d'une capsule *leadless.*
La Figure 3 est une vue en perspective d'un premier mode de réalisation du corps d'une capsule implantable selon l'invention.
La Figure 4 est une vue en perspective d'un deuxième mode de réalisation du corps d'une capsule implantable selon l'invention.
La Figure 5a est une vue en coupe d'un transducteur piézoélectrique au repos d'une capsule implantable selon l'invention.
La Figure 5b est une vue du transducteur de la Figure 5a en fonctionnement.
La Figure 5c est une vue partielle en perspective du transducteur des Figures 5a et 5b.
Les Figures 6a à 6c sont des vues de dessus de composants piézoélectriques formés de bandes rectilignes.
Les Figures 7a et 7b sont des vues de dessus de composants piézoélectriques formés de bandes enroulées en spirale.
La Figure 8 est une vue de dessus d'un composant piézoélectrique de structure annulaire.
Les Figures 9a à 9c sont des vues en perspective de différentes structures de bandes piézoélectriques rectilignes.
Les Figures 10a à 10c sont des vues en perspective de différentes structures d'électrodes pour des bandes piézoélectriques unimorphes.
Les Figures 11a à 11c sont des vues en perspective de différentes structures d'électrodes pour des bandes piézoélectriques bimorphes.
Les Figures 12a et 12b sont des vues en coupe montrant respectivement des configurations série et parallèle d'électrodes de la structure de la Figure 11a.
Les Figures 13a et 13b sont des vues en perspective montrant des transducteurs piézoélectriques constitués respectivement de composants piézoélectriques rectilignes et spiralés en série.
Les Figures 14a et 14b sont des vues en perspective montrant des transducteurs piézoélectriques constitués respectivement de composants piézoélectriques rectilignes et spiralés en parallèles.
La Figure 15 est une vue en coupe d'une première variante du transducteur de la Figure 5a.
La Figure 16 est une vue en coupe d'une deuxième variante du transducteur de la Figure 5a.
La Figure 17a est un exemple de diagramme force F-déplacement u du cycle de fonctionnement d'une capsule implantable conforme à l'invention.
La Figure 17b est un exemple de diagramme tension *V*-charge Q du cycle de fonctionnement d'une capsule implantable conforme à l'invention.

On va maintenant décrire divers exemples de réalisation du transducteur de récupération d'énergie selon l'invention.

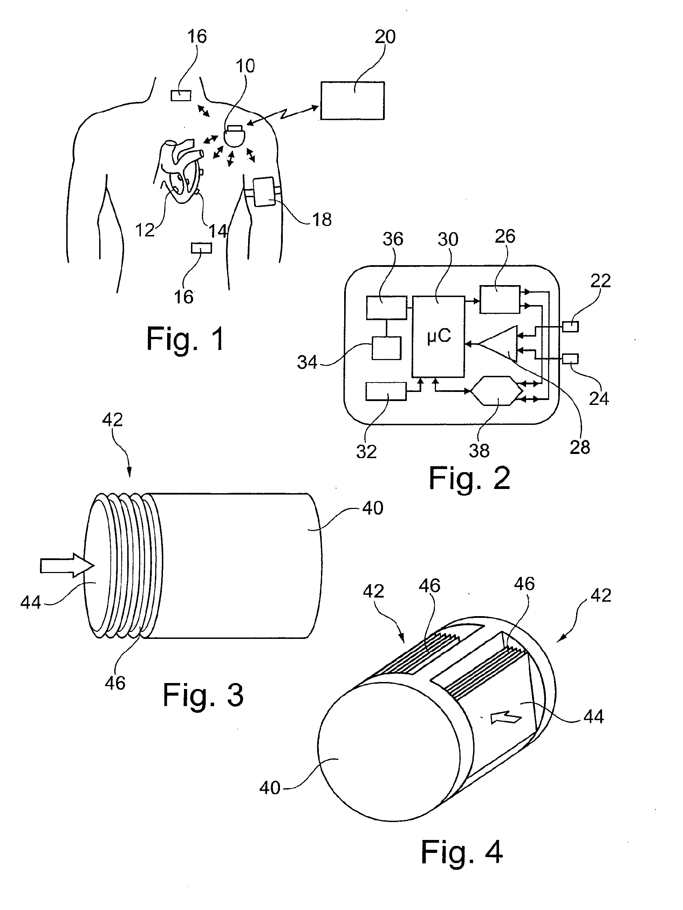

Sur la Figure 1 est illustré un ensemble de dispositifs médicaux implantés au sein du corps d'un patient.

Celui-ci est équipé par exemple d'un implant 10 tel qu'un défibrillateur/ stimulateur/resynchroniseur implanté, un défibrillateur sous-cutané ou encore un enregistreur longue durée. Ce dispositif 10 est le dispositif maître d'un réseau comportant une pluralité de dispositifs esclaves 12 à 18, qui peuvent notamment inclure des capsules intracardiaques 12 ou épicardiques 14 implantées directement sur le coeur du patient, d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif 10 peut également être utilisé en tant que passerelle avec le monde extérieur pour communiquer avec un périphérique externe 20 du type programmateur ou dispositif de télétransmission de données avec lequel il pourra communiquer par télémétrie.

La Figure 2 illustre de façon schématique les différents circuits internes des capsules autonomes implantées 12 à 16.

La capsule comporte par exemple un couple d'électrodes 22, 24 reliées à un circuit 26 générateur d'impulsions de stimulation (pour une capsule active incorporant cette fonction) et/ou à un circuit de détection 28 servant au recueil des potentiels de dépolarisation recueillis entre les électrodes 22 et 24. Un circuit central 30 inclut l'ensemble de l'électronique permettant de piloter les diverses fonctions de la capsule, la mémorisation des signaux recueillis, etc. Il comprend un microcontrôleur et un oscillateur générant les signaux d'horloge nécessaires au fonctionnement du microcontrôleur et à la communication. Il peut également contenir un convertisseur analogique/numérique et une mémoire de stockage numérique. La capsule peut également être pourvue d'un capteur 32 tel qu'un capteur d'accélération, de pression, un capteur hémodynamique, de température, de saturation en oxygène, etc. La capsule comprend un étage de récupération d'énergie 34 alimentant l'ensemble des circuits via un étage de gestion d'énergie 36. Les électrodes 22 et 24 sont également reliées à un circuit d'émission/réception d'impulsions 38 servant à la communication sans fil avec le dispositif maître ou les autres capsules.

L'invention concerne plus particulièrement l'étage de récupération d'énergie 34 qui, de façon caractéristique, utilise les variations de pression du milieu environnant, notamment les variations cycliques de la pression sanguine, pour déformer un matériau piézoélectrique. La récupération d'énergie est obtenue grâce à la création de charges électriques résultant des contraintes mécaniques de déformation appliquées au matériau piézoélectrique sous l'effet des variations de pression sanguine.

Pour pouvoir prendre en compte ces déformations, la capsule est réalisée sous forme d'un corps 40 pourvu, comme illustré Figures 3 et 4, d'un ou plusieurs éléments déformables 42 sollicités au rythme des variations de la pression du fluide dans lequel baigne la capsule, typiquement les variations de pression sanguine, dans le cas d'une capsule cardiaque. L'élément déformable 42 comprend une surface rigide 44 sur laquelle s'exerce la pression, et qui est reliée au reste du corps par l'intermédiaire d'un soufflet 46 déformable sous l'effet de la sollicitation externe à laquelle est soumise la surface rigide 44.

Dans l'exemple de la Figure 3, cet ensemble surface/soufflet 44, 46 est disposé sur une face d'extrémité axiale du corps 40 de la capsule, qui présente une forme générale cylindrique. Les dimensions sont typiquement de l'ordre de 6 mm de diamètre pour une longueur de 20 mm, soit un très faible volume de l'ordre de 0,5 cm³.

Dans l'exemple de la Figure 4, il est prévu deux ensembles déformables 42 disposés sur des faces latérales du corps 40 de la capsule, les surfaces rigides 44, reliées au bloc 40 par le soufflet 46, étant des surfaces parallèles entre elles et à l'axe principal de la capsule. Cette configuration permet notamment de dédoubler le système de récupération d'énergie ; elle libère en outre les deux extrémités axiales de la capsule, ce qui peut avoir son importance notamment pour y placer une vis d'ancrage sans que le système de récupération d'énergie ne fasse obstacle à cette configuration.

Dans l'un ou l'autre cas, le corps 40 avec son élément déformable 42 sont avantageusement réalisés sous forme monobloc, par exemple en titane évaporé ou électrodéposé sur un mandrin soluble.

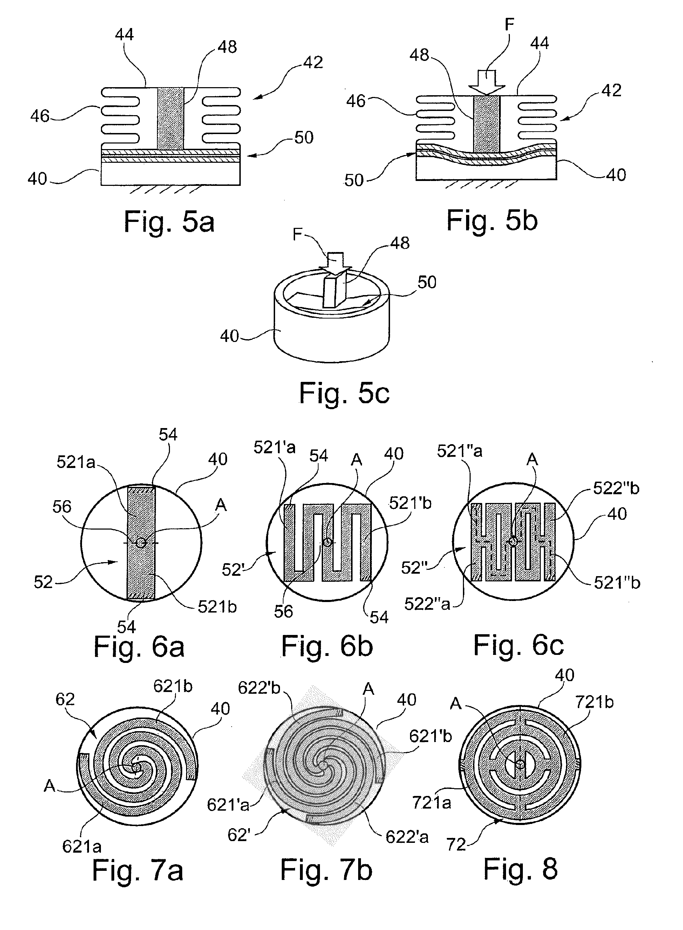

Sur l'exemple de réalisation des Figures 5a à 5c, on peut voir que la sollicitation physique extérieure F de pression sanguine appliquée à la surface rigide 44 est transmise via une tige 48 de liaison à un transducteur 50 de récupération d'énergie du type piézoélectrique, apte à convertir cette sollicitation mécanique F en charges électriques grâce à l'effet piézoélectrique direct selon lequel la sollicitation mécanique extérieure créée engendre la création de charges électriques aux bornes d'électrodes déposées dans le transducteur 50 de part et d'autre d'un matériau piézoélectrique. L'énergie électrique ainsi récupérée est ensuite traitée par le module 36 de stockage et de gestion d'énergie.

D'une manière générale, l'énergie mécanique en entrée est due à la force de pression sanguine de faible intensité, à savoir quelques dizaines à quelques centaines de mN pour un grand déplacement, de l'ordre de quelques centaines de µm. Cela implique que la raideur du système doit être faible, typiquement quelques centaines à quelques milliers de mN/m. Or ce genre de raideur est très difficile à obtenir avec des éléments piézoélectriques standard tels que ceux décrits dans l'article de l'état de la technique précité.

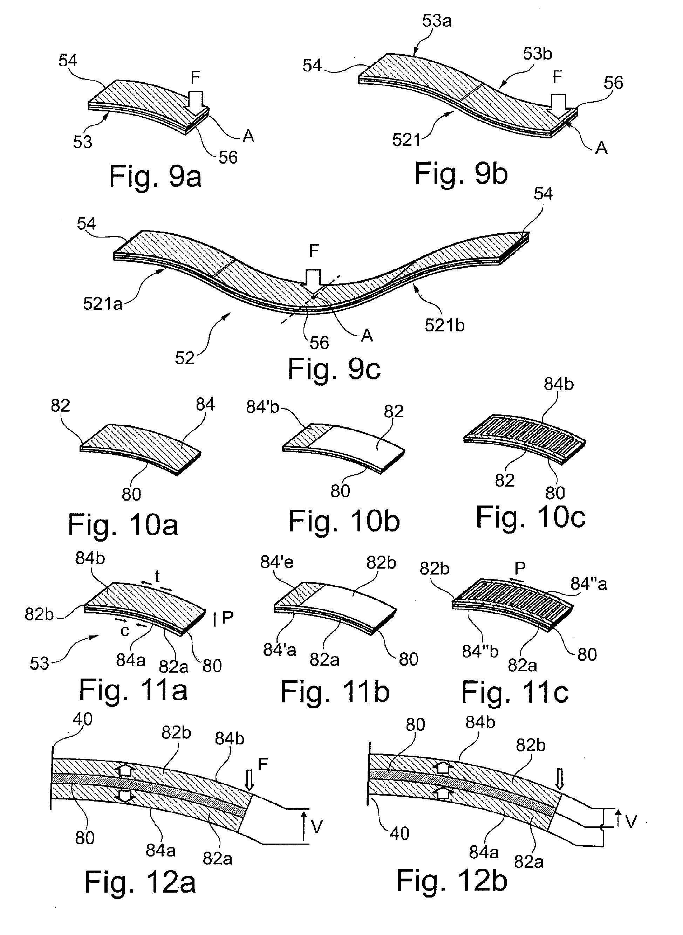

Pour répondre à ces critères de souplesse et dimensions, tout en restant compatible avec l'exigence de miniaturisation, il est proposé que les composants piézoélectriques constituant les transducteurs d'énergie mis en oeuvre dans des capsules conformes à l'invention présentent une structure comprenant au moins une bande qui, à l'image de la bande 53 de la Figure 9a, est équivalente à une poutre simplement encastrée fine et longue, fixée à une extrémité 54 au corps 40 de la capsule et soumise à la sollicitation extérieure F en un point A d'application situé à une autre extrémité 56. Les structures visées ont, de préférence, quelques millimètres de longueur, quelques centaines de micromètres de largeur et quelques dizaines à quelques centaines de micromètres d'épaisseur. Comme on peut le voir sur la Figure 11a, la bande 53 a une structure électrique bimorphe avec deux couches piézoélectriques 82a, 82b disposées de part et d'autre d'un substrat 80 et deux électrodes 84a, 84b couvrant entièrement les couches piézoélectriques. Dans ce cas, le composant est polarisé selon l'épaisseur de la bande 53 pour une récupération en mode 31 dans lequel la polarisation P est perpendiculaire à la direction de la contrainte, à savoir traction T sur la couche supérieure 82b et compression C sur la couche inférieure 82a. De plus, ainsi que l'indiquent les Figures 12a et 12b, les électrodes 84a, 84b peuvent être montées en série (Figure 12a) ou en parallèle (Figure 12b).

Les couches piézoélectriques 82a, 82b peuvent être réalisées en un matériau comme la céramique PZT ou des monocristaux de type PMN-PT, titanate de baryum ou niobate de lithium présentant un fort couplage électromécanique.

Sur la Figure 11b, les électrodes 84'a, 84'b ne recouvrent que partiellement les couches piézoélectriques 82a, 82b, à l'endroit où les contraintes sont maximales, à proximité de l'encastrement dans le corps 40 la capsule. La polarisation et le mode de fonctionnement sont similaires au cas de la Figure 11 a.

Selon le mode de réalisation de la Figure 11c, le composant piézoélectrique fonctionne selon le mode 33 dans lequel la polarisation P est parallèle à la contrainte, c'est-à-dire le long de la bande, car le couplage électromécanique y est le plus fort. Les électrodes 84"a, 84"b sont alors conformées de manière interdigitée.

Les Figures 10a à 10c montrent des structures de composants similaires à celles des Figures 11a à 11c, appliquées à des composants unimorphes ne comportant qu'une seule couche piézoélectrique 82 sur une face du substrat 80.

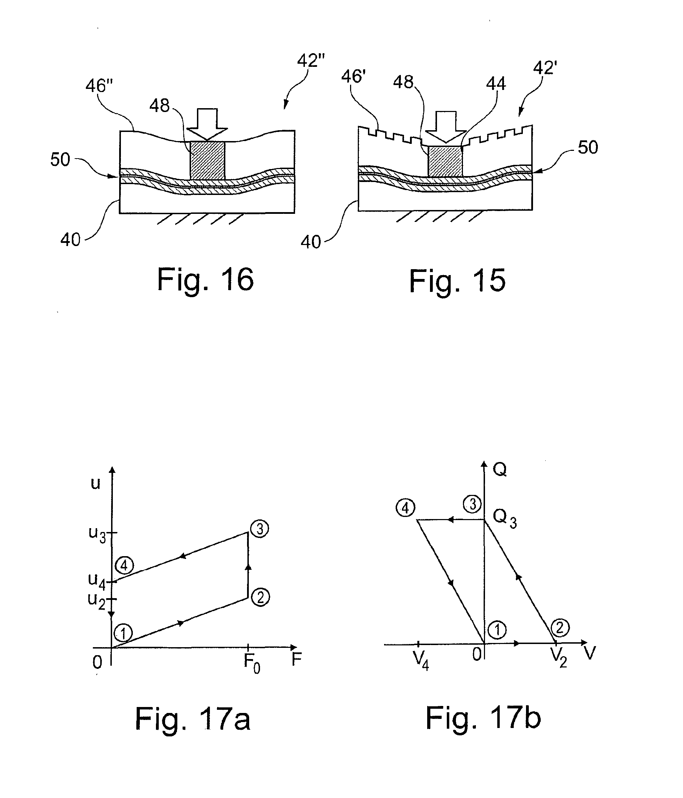

On va maintenant décrire en regard des Figures 17a et 17b un exemple de cycle de conversion d'énergie au cours duquel la force F appliquée au transducteur 50 passe d'une valeur nulle à une valeur maximale F₀. Initialement, en position ①, le système est au repos et toutes les variables sont nulles.

De la position ① à la position ②, le circuit est ouvert et la force appliquée F croît jusqu'à F₀. Lors de cette phase, la charge Q reste nulle et la tension V passe de 0 à V₂.

De la position ② à la position ③, sous la force F₀, on ferme le circuit en récupérant les charges accumulées. En ③, on a donc Q = Q₃. Le circuit est ensuite réouvert.

De la position ③ à la position ④, la force F revient à 0 et la charge reste constante, à Q₃. En ④, V₄ = -V₂.

De la position ④ à la position ①, on ferme le circuit en récupérant une deuxième fois la charge.

L'énergie récupérée par cycle est donc W = V₂Q₃.

D'autre cycles de conversion sont également possibles, comme par exemple un cycle à une seule extraction de charge (passant directement de l'étape ③ à l'étape ①), ou par exemple un cycle où la tension est imposée aux bornes de la couche piézoélectrique pour optimiser l'extraction de l'énergie.

La Figure 9b illustre une autre structure de bande 521 en poutre simplement encastrée, formée de deux bandes 53a, 53b analogues à la bande 53 de la Figure 9a, disposées en série et dont les concavités sont de signe contraires. Les polarités développées par ces bandes étant opposées, les électrodes de chacune des bandes sont isolées électriquement les unes des autres.

Sur la Figure 9c est représenté le composant 52 des Figures 5c et 6a. Ce composant est équivalent à une poutre doublement encastrée résultant de la mise en parallèle de deux composants simplement encastrées 521 a, 521b identiques à celui de la Figure 9b. Dans ce cas également, l'inversion de polarité le long de la poutre impose l'isolement électrique des électrodes en fonction de leur concavité.

Les Figures 6b à 8 montrent d'autres composants piézoélectriques constitués en divers agencements de bandes ayant pour but d'allonger la longueur de la structure piézoélectrique sans perdre en compacité.

Les bandes des composants 52', 52" des Figures 6b et 6c sont formés de segments rectilignes repliés.

Le composant piézoélectrique 52' de la Figure 6b comprend deux bandes 521'a, 521'b disposées en parallèle de manière analogue aux bandes 521 a, 521 b de la Figure 6a.

Le composant piézoélectrique 52" de la Figure 6c est plus complexe car il comprend quatre bandes 521"a, 521"b, 522"a, 522"b en parallèle deux à deux pour former deux composants du type de celui de la Figure 6b, eux-mêmes disposés en parallèle.

Le composant piézoélectrique 62 de la Figure 7a est équivalent au composant 52' de la Figure 6b, la différence portant sur le fait que les deux bandes 621 a, 621 b en parallèle sont enroulées en spirales.

De même, le composant piézoélectrique 62' de la Figure 7b est équivalent au composant 52" de la Figure 6c au sens où il s'agit d'une structure à quatre bandes 621'a, 621'b, 622'a, 622'b enroulées en spirales formant deux composants en parallèle constitués, d'une part, des bandes 621'a, 621'b en parallèle et, d'autre part, des bandes 622'a, 622'b en parallèle. Enfin, le composant 72 de la Figure 8 est équivalent à une poutre doublement encastrée formée de deux structures 721 a, 721 b en parallèle de bandes annulaires.

En outre, il est critique dans le cas d'un récupérateur d'énergie piézoélectrique de limiter les contraintes mécaniques pour avoir un système fiable, sans fatigue sur un grand nombre de cycles, typiquement à moins de quelques dizaines de MPa pour un matériau de type PZT, et de limiter la tension aux bornes du composant piézoélectrique afin de la rendre compatible avec des systèmes électroniques classiques, c'est à dire moins de 15 ou 20 V.

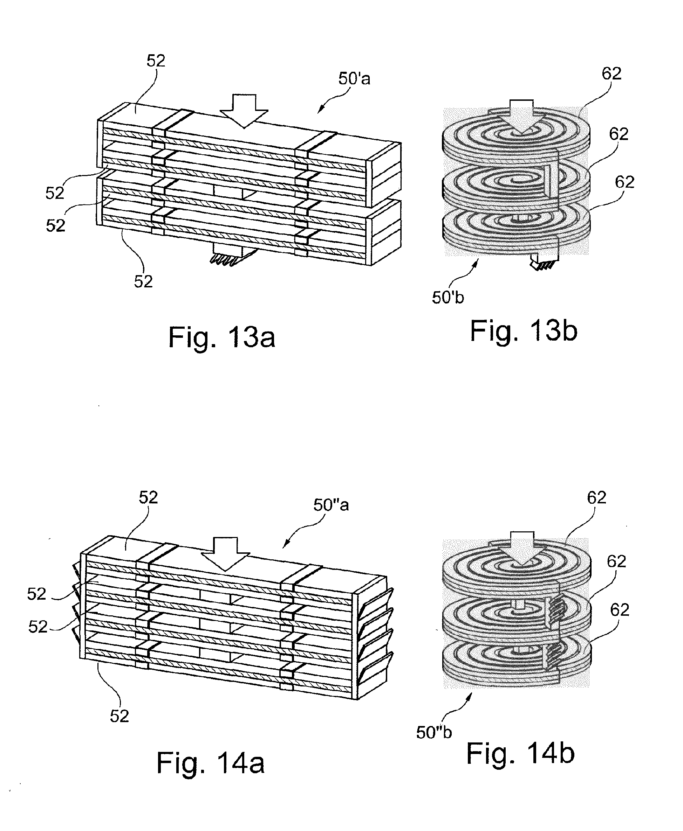

Dans ce but, il est proposé de réaliser les transducteurs de récupération en agençant en série et en parallèle plusieurs composants piézoélectriques, par exemple de type poutre ou spirale. En effet, pour une énergie mécanique en entrée donnée par une force et un déplacement, mettre des structures en série va réduire les déformations de chaque élément et ainsi réduire les contraintes mécaniques, et mettre des structures en parallèle permet de diminuer l'épaisseur des composants piézoélectriques pour abaisser la tension à champ électrique constant dans le matériau. Les Figures 13a et 13b montrent des transducteurs 50'a, 50'b constitués respectivement de composants de type poutre 52 et de type spirale 62 assemblés en série.

De même, les Figures 14a et 14b montrent des transducteurs 50"a et 50"b résultant de l'assemblage en parallèle de ces mêmes composants.

La Figure 15 représente un deuxième mode de réalisation d'une capsule selon l'invention, dans lequel l'élément mobile 42' d'actionnement comprend une surface rigide 44 plane couplée au corps 40 de la capsule par un organe élastiquement déformable 46' de liaison réalisé sous forme d'ondulations périphériques autour de la surface rigide 44.

La Figure 16 représente un troisième mode de réalisation, avec un élément mobile 42" d'actionnement constitué d'une membrane souple étirable 46" fixée au corps 40 de la capsule à sa périphérie et portant en son centre la tige 48 de liaison au transducteur 50 de récupération d'énergie.

## Revendications

1. Une capsule intracorporelle autonome, comportant un corps (40) et, à l'intérieur de ce corps, des circuits électroniques (26-36) et des moyens d'alimentation électrique dépourvus de masse sismique, comprenant :
- un transducteur (50) de récupération d'énergie, apte à convertir une sollicitation physique extérieure (F) appliquée à la capsule en grandeur électrique, ce transducteur comprenant au moins un composant piézoélectrique (52) couplé à un élément mobile (42) d'actionnement recevant ladite sollicitation physique extérieure,
ce composant piézoélectrique (52) présentant au moins une structure à au moins une bande piézoélectrique (521a, 521b) fixée au corps (40) de la capsule à une extrémité (54) et apte à être soumise à une sollicitation physique extérieure (F) en un point d'application (A) situé à une autre extrémité (56) ; et
- un module (36) de stockage et de gestion d'énergie, alimenté par le transducteur (50) de récupération d'énergie sous l'effet d'une déformation du composant piézoélectrique (52) due à la sollicitation physique extérieure (F) transmise par l'élément mobile (42) d'actionnement,
**caractérisée en ce que** l'élément mobile (42) d'actionnement comprend une surface rigide (44) couplée au composant piézoélectrique et un soufflet élastiquement déformable (46) de liaison de cette surface rigide au reste du corps (40).

2. La capsule de la revendication 1, dans laquelle la bande piézoélectrique (521 a, 521 b) est de forme rectiligne.

3. La capsule de la revendication 1, dans laquelle la bande piézoélectrique (621 a, 621 b) est enroulée en spirale.

4. La capsule de la revendication 1, dans laquelle la bande piézoélectrique (521'a, 521'b) est formée de segments rectilignes repliés.

5. La capsule de la revendication 1, dans laquelle la bande piézoélectrique (721 a, 721 b) a une structure annulaire.

6. La capsule de la revendication 1, dans laquelle le composant piézoélectrique (52 ; 52' ; 62) comprend deux bandes piézoélectriques en parallèle autour d'un point (A) d'application commun.

7. La capsule de la revendication 6, dans laquelle le composant piézoélectrique (52" ; 62') présente une pluralité de structures de bandes disposées en parallèle.

8. La capsule de la revendication 1, dans laquelle le transducteur (50'a ; 50'b) de récupération d'énergie comporte une pluralité de composants piézoélectriques (52 ; 62) disposés en série.

9. La capsule de la revendication 1, dans laquelle le transducteur (50"a ; 50"b) de récupération d'énergie comporte une pluralité de composants piézoélectriques (52 ; 62) disposés en parallèle.

10. La capsule de la revendication 1, dans laquelle la bande piézoélectrique (521 a, 521 b) est formée d'un substrat (80), d'au moins une couche de matériau piézoélectrique (82a, 82b) déposée sur une face du substrat et d'au moins une électrode (84a, 84b) couvrant au moins partiellement la couche de matériau piézoélectrique.

11. La capsule de la revendication 10, dans laquelle une couche (82) de matériau piézoélectrique est déposée sur une face du substrat (80).

12. La capsule de la revendication 10, dans laquelle une couche (82a, 82b) de matériau piézoélectrique est déposée sur chaque face du substrat (80).

13. La capsule de la revendication 10, dans laquelle l'électrode (84 ; 84a, 84b) couvre totalement la couche (82 ; 82a, 82b) de matériau piézoélectrique.

14. La capsule de la revendication 10, dans laquelle la au moins une électrode (84' ; 84'a, 84'b) couvre partiellement la couche (82 ; 82a, 82b) de matériau piézoélectrique.

15. La capsule de la revendication 10, dans laquelle les électrodes (84" ; 84"a, 84"b) couvrant la couche (82 ; 82a, 82b) de matériau piézoélectrique sont interdigitées.

## Patentansprüche

1. Autonome intrakorporale Kapsel, die einen Körper (40) und im Inneren dieses Körpers elektronische Schaltkreise (26-36) und Stromversorgungseinrichtungen ohne seismische Masse aufweist, die enthält:
- einen Energierückgewinnungswandler (50), der eine auf die Kapsel angewendete äußere physikalische Beanspruchung (F) in eine elektrische Größe umwandeln kann, wobei dieser Wandler mindestens ein piezoelektrisches Bauteil (52) enthält, das mit einem beweglichen Aktivierungselement (42) gekoppelt ist, welches die äußere physikalische Beanspruchung empfängt,
wobei dieses piezoelektrische Bauteil (52) mindestens eine Struktur mit mindestens einem piezoelektrischen Streifen (521a, 521b) enthält, der an einem Ende (54) am Körper (40) der Kapsel befestigt ist und an einem an einem anderen Ende (56) befindlichen Anwendungspunkt (A) einer äußeren physikalischen Beanspruchung (F) ausgesetzt werden kann; und
- ein Modul (36) zum Speichern und zur Verwaltung von Energie, das vom Energierückgewinnungswandler (50) unter der Wirkung einer Verformung des piezoelektrischen Bauteils (52) aufgrund der äußeren physikalischen Beanspruchung (F) gespeist wird, die vom beweglichen Aktivierungselement (42) übertragen wird,
**dadurch gekennzeichnet, dass** das bewegliche Aktivierungselement (42) eine mit dem piezoelektrischen Bauteil gekoppelte steife Fläche (44) und einen elastisch verformbaren Faltenbalg (46) zur Verbindung dieser steifen Fläche mit dem Rest des Körpers (40) enthält.

2. Kapsel nach Anspruch 1, wobei der piezoelektrische Streifen (521a, 521b) von geradliniger Form ist.

3. Kapsel nach Anspruch 1, wobei der piezoelektrische Streifen (621a, 621b) spiralförmig gewickelt ist.

4. Kapsel nach Anspruch 1, wobei der piezoelektrische Streifen (521'a, 521'b) von gefalteten geradlinigen Segmenten geformt wird.

5. Kapsel nach Anspruch 1, wobei der piezoelektrische Streifen (721a, 721b) eine ringförmige Struktur hat.

6. Kapsel nach Anspruch 1, wobei das piezoelektrische Bauteil (52; 52'; 62) zwei piezoelektrische Streifen parallel um einen gemeinsamen Anwendungspunkt (A) herum enthält.

7. Kapsel nach Anspruch 6, wobei das piezoelektrische Bauteil (52"; 62') eine Vielzahl von Strukturen von parallel angeordneten Streifen aufweist.

8. Kapsel nach Anspruch 1, wobei der Energierückgewinnungswandler (50'a; 50'b) eine Vielzahl von in Reihe angeordneten piezoelektrischen Bauteilen (52; 62) aufweist.

9. Kapsel nach Anspruch 1, wobei der Energierückgewinnungswandler (50"a; 50"b) eine Vielzahl von parallel angeordneten piezoelektrischen Bauteilen (52; 62) aufweist.

10. Kapsel nach Anspruch 1, wobei der piezoelektrische Streifen (521a, 521b) von einem Substrat (80), mindestens einer Schicht von piezoelektrischem Material (82a, 82b), das auf eine Seite des Substrats aufgebracht ist, und mindestens einer Elektrode (84a, 84b) gebildet wird, die die Schicht aus piezoelektrischem Material zumindest teilweise bedeckt.

11. Kapsel nach Anspruch 10, wobei eine Schicht (82) aus piezoelektrischem Material auf eine Seite des Substrats (80) aufgebracht wird.

12. Kapsel nach Anspruch 10, wobei eine Schicht (82a, 82b) aus piezoelektrischem Material auf jede Seite des Substrats (80) aufgebracht wird.

13. Kapsel nach Anspruch 10, wobei die Elektrode (84;
84a, 84b) die Schicht (82; 82a, 82b) aus piezoelektrischen Material ganz bedeckt.

14. Kapsel nach Anspruch 10, wobei die mindestens eine Elektrode (84'; 84'a, 84'b) die Schicht (82; 82a, 82b) aus piezoelektrischem Material teilweise bedeckt.

15. Kapsel nach Anspruch 10, wobei die die Schicht (82; 82a, 82b) aus piezoelektrischem Material bedeckenden Elektroden (84"; 84"a, 84"b) ineinander verzahnt sind.

## Claims

1. An autonomous intracorporeal capsule, including a body (40) and, inside this body, electronic circuits (26-36) and electric supply means devoid of seismic mass, comprising:
- an energy harvesting transducer (50), adapted to convert an external physical stress (F) applied to the capsule into an electrical quantity, this transducer comprising at least one piezoelectric component (52) coupled to a mobile operating element (42) receiving said external physical stress,
this piezoelectric component (52) having at least one structure with at least one piezoelectric band (521a, 521b) fixed to the body (40) of the capsule at one end (54) and adapted to be subjected to an external physical stress (F) at an application point (A) located at another end (56); and
- an energy storage and management module (36), supplied by the energy harvesting transducer (50) under the effect of a deformation of the piezoelectric component (52) due to the external physical stress (F) transmitted by the mobile operating element (42),
**characterized in that** the mobile operating element (42) comprises a rigid surface (44) coupled to the piezoelectric component and an elastically deformable bellows (46) for the connection of this rigid surface to the remaining of the body (40).

2. The capsule of claim 1, wherein the piezoelectric band (521a, 521b) is rectilinear in shape.

3. The capsule of claim 1, wherein the piezoelectric band (621a, 621b) is wound into a spiral.

4. The capsule of claim 1, wherein the piezoelectric band (521'a, 521'b) is formed of folded rectilinear segments.

5. The capsule of claim 1, wherein the piezoelectric band (721a, 721b) has an annular structure.

6. The capsule of claim 1, wherein the piezoelectric component (52; 52'; 62) comprises two piezoelectric bands in parallel about a common point of application (A).

7. The capsule of claim 6, wherein the piezoelectric component (52"; 62') has a plurality of band structures arranged in parallel.

8. The capsule of claim 1, wherein the energy harvesting transducer (50'a; 50'b) includes a plurality of piezoelectric components (52; 62) arranged in series.

9. The capsule of claim 1, wherein the energy harvesting transducer (50"a; 50"b) includes a plurality of piezoelectric components (52; 62) arranged in parallel.

10. The capsule of claim 1, wherein the piezoelectric band (521a, 521b) is formed of a substrate (80), at least one layer (82a, 82b) of piezoelectric material deposited on a face of the substrate and at least one electrode (84a, 84b) covering at least partially the layer of piezoelectric material.

11. The capsule of claim 10, wherein a layer (82) of piezoelectric material is deposited on one face of the substrate (80).

12. The capsule of claim 10, wherein a layer (82a, 82b) of piezoelectric material is deposited on each face of the substrate (80).

13. The capsule of claim 10, wherein the electrode (84; 84a, 84b) covers fully the layer (82; 82a, 82b) of piezoelectric material.

14. The capsule of claim 10, wherein the at least one electrode (84'; 84'a, 84'b) covers partially the layer (82; 82a, 82b) of piezoelectric material.

15. The capsule of claim 10, wherein the electrodes (84"; 84"a, 84"b) covering the layer (82; 82a, 82b) of piezoelectric material are interdigited.
